# EUROPÄISCHE PATENTSCHRIFT

(11) **EP 0 643 036 B1**
(45) Veröffentlichungstag und Bekanntmachung des Hinweises auf die Patenterteilung: **04.06.1997**
(21) Anmeldenummer: 94111105.6
(22) Anmeldetag: 16.07.1994
(51) Int. Cl.: C07C 67/00, C07C 69/34

(54) **Verfahren zur Herstellung von Pimelinsäureestern**
Process for the preparation of esters of pimelic acid
Procédé de préparation d'esters d'acide pimélique

(30) Priorität: 13.09.1993 DE 4331000
(43) Veröffentlichungstag der Anmeldung: 15.03.1995
(73) Patentinhaber: HÜLS AKTIENGESELLSCHAFT, 45764 Marl (DE)
(72) Erfinder: Köhler, Dr. Günther, D-45772 Marl (DE)

(56) Entgegenhaltungen:
- DE-A- 1 668 730

## Beschreibung

Die Erfindung betrifft ein neues Verfahren zur Herstellung von Pimelinsäureestern, insbesondere durch Umsetzung von Cyclohexanon mit Dialkylcarbonat in Gegenwart von Alkalialkoholaten und anschließende Reaktion mit Alkoholen in einem Reaktionsgefäß. Pimelinsäureester im Sinne des Verfahrens haben die folgende Formel: 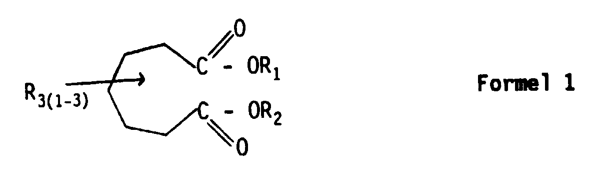 R₁ und R₂ können gleich oder verschieden sein. R₁ oder R₂ sind gesättigte Alkylsubstituenten von CH₃- bis C₁₀H₂₁-. Sie können aber auch andere Substituenten, wie Araliphaten mit 7 bis 10 C-Atomen, z. B. Benzyl, Phenylethyl, sein. R₃ kann H, ein KW-Rest von C₁-C₆-Atomen sein, insbesondere CH₃-.

Pimelinsäureester sind wichtige Zwischenprodukte zur Herstellung von Pharmaka, Pflanzenschutzmitteln, Riechstoffen oder kosmetischen Wirkstoffen, Spezialpolymeren sowie Schmierstoffen.

Aus der DE-A-16 68 730 ist bekannt, Cycloalkanone mit Dialkylcarbonaten in Gegenwart von Alkoholaten oder Alkalimetallen um zu setzen. Dabei werden nach Aufarbeitung mit wäßrigen Mineralsäuren alpha, omega-Dicarbonsäurediester hergestellt.

Es ist bekannt, daß Pimelinsäure durch partielle Hydrierung von Salicyl-Säure - zu Tetrahydrosalicylsäure - und anschließende Aufspaltung von Natrium- oder Kaliumhydroxid unter drastischen Bedingungen hergestellt werden kann, vgl. *Ullmann*, *Band 5*, *S*. *824 (1954) und Band 10*, *S*. *140 (1980)* und *Org*. *Synthesis Coll*. *II*, *S*. *531-38 (1943)*. Gleichfalls durch Alkalibehandlung kann Pimelinsäure auch aus Cyclohex-4-encarbonsäure gewonnen werden, die ihrerseits aus Butadien und Acrylsäure durch Diels-Alder zugänglich ist [*J*. *Amer*. *Chem*. *Soc*. *74*, *S*. *532* (*1952)]*.

Auch durch Umsetzung von Alkali bzw. Alkalihydroxiden mit Tetrahydrobenzaldehyd gemäß *DBP 7331* oder mit Tetrahydrofurylpropanol gemäß *DBP 2939* kann Pimelinsäure hergestellt werden.

Nach dem Verfahren der *EP-A 0 905 651* kann die Synthese auch durch Reduktion von 3-Oxopimelinsäureester erfolgen.

Weitere Synthesewege gehen von Suberon aus, das durch oxidative Spaltung in Pimelinsäure überführt wird, vgl. *US-PS 286 516* und *DE-A 20 43 012*. Zum gleichen Ergebnis gelangt man, indem Cyclohexanon-2-carbonsäureamid verseift wird, vgl. *DD-PS 112 987*.

Die Oxidation von Cycloalkenen mit Ozon nach *DE-A 40 00 163* sowie die Hydroformylierung von 1,5-Pentadien und anschließende Oxidation nach *DE-A 15 18 216* oder Carbonylierung von ω-Caprolacton nach *US-PS 4 888 443* führt gleichfalls zu Pimelinsäure.

Für viele Anwendungszwecke sind allerdings die Pimelinsäureester wünschenswert, die im Anschluß an die bisher beschriebenen Synthesen durch Veresterung nach bekannten Methoden aus der Pimelinsäure hergestellt werden müssen. Diese oft mehrstufigen Synthesen sowie die anschließende Veresterung sind zur Gewinnung von Pimelinsäureestern sehr aufwendig. Alle bekannten Verfahren sind zudem aus technischer Sicht sehr unbefriedigend.

Es wurde nun gefunden, daß durch Umsetzung von Salzen aus 2-Oxocyclohexancarbonsäureestern und starken organischen Basen mit Alkoholen bei erhöhten Temperaturen unter Ringöffnung direkt Dialkylpimelate mit hoher Selektivität und Ausbeute gebildet werden. Im Vergleich zu bekannten Verfahren liefert der Syntheseweg nach der Erfindung auf einfachem und wirtschaftlichem Weg direkt Dialkylester der Pimelinsäure nach Reaktionsgleichung I und II.

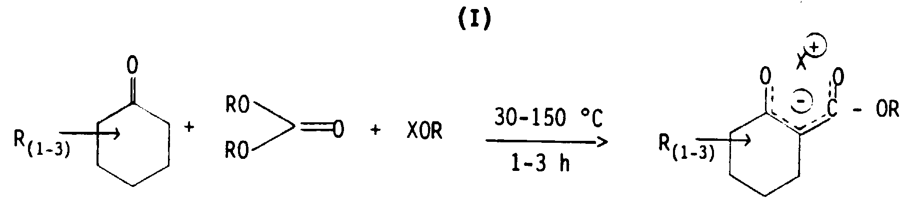

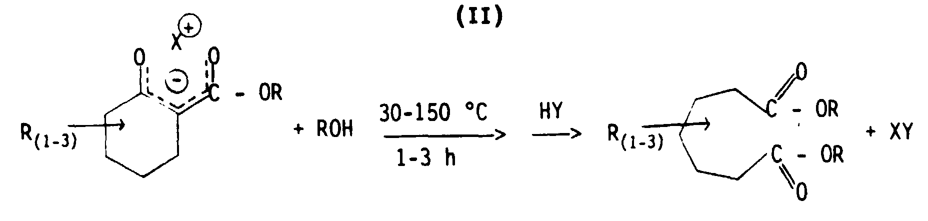

Überraschend und bisher nicht bekannt ist, daß ein bei dieser Synthese als Zwischenprodukt auftretendes und üblicherweise nicht isoliertes Salz einer starken Base, der aciden β-Ketoesterverbindung des Cyclohexanons - das vorzugsweise ein Alkalisalz, z. B. von Na, K, sein kann - mit einem Alkohol zum Pimelinsäureester gespalten wird.

Gegenstand der Erfindung ist ein Verfahren zur Herstellung von 0 bis 3 Alkylsubstituenten enthaltenden Pimelinsäureestern aliphatischer oder aromatischer Alkohole, welches dadurch gekennzeichnet ist, daß in an sich bekannter Weise ein gegebenenfalls alkylsubstituiertes Cyclohexanon zu dem entsprechenden 2-Oxocyclohexancarbonsäureester carboxyliert wird und gleichzeitig oder anschließend in Gegenwart einer starken Base das entsprechende Salz gebildet wird, welches mit einem Alkohol bei Temperaturen von 50 - 250 °C unter Ringöffnung umgesetzt wird.

Die Synthese der 2-Oxocyclohexancarbonsäureester (Gleichung I) geschieht nach bekannten Verfahren, indem z. B. das bekannte und preiswerte Edukt Cyclohexanon mit Dialkylcarbonat in Gegenwart von Alkalibasen carboxyliert wird.

Es ist dabei vorteilhaft, ein Lösungsmittel bei der Synthese einzusetzen, um das Reaktionsgemisch gut rührbar zu halten. Als Lösungsmittel eignen sich z. B. Toluol, Cyclohexan, tert.-Butylmethylether, Xylol, Mesitylen, tert.-Butylbenzol, p.-tert.-Butyltoluol, aber auch polare Lösemittel, wie Anisol, Alkylanisol, Phenoxyethanol, Benzylalkohol, oder 3,3,5-Trimethylcyclohexanon oder Dimethylcarbonat, Diethylcarbonat, die gleichzeitig auch Reaktionspartner sein können.

Die erfindungsgemäße Reaktion nach Gleichung II findet erst nach Beendigung der Reaktion Gleichung I statt bzw. kann erst eingeleitet werden, wenn das Salz aus einem Oxocyclohexancarbonsäureester mit einer starken Base gebildet wurde.

Verwendet man niedere Alkohole, wie z. B. Methanol, Ethanol oder Propanol, kann die Reaktion auch unter Normaldruck bei Siederückfluß stattfinden. Vorteilhafter bzw. vollständig vollzieht sich die Ringöffnung jedoch bei erhöhter Temperatur ab 90 °C bis 250 °C innerhalb von 10 bis 30 Minuten, wobei unter leicht erhöhtem Druck gearbeitet wird. Bei Verwendung höherer Alkohole, deren Siedepunkte oberhalb von 90 °C liegen, kann dieser Vorgang auch drucklos unter Siederückfluß erfolgen.

Die Ringöffnung nach Gleichung II kann allerdings auch mit niederen Alkoholen, wie Methanol, Ethanol oder Propanol, bei höheren Temperaturen, wie z. B. 120 °C, unter Normaldruck ausgeführt werden, wenn der niedere Alkohol so langsam zudosiert wird, daß sofort die Ringöffnung unter Verbrauch des Alkohols erfolgt.

Wird die Behandlung des 2-Oxocyclohexancarbonsäureesters mit einem anderen bzw. höheren Alkohol als der des Alkoholrestes im 2-Oxocyclohexancarbonsäureester ausgeführt, so werden gemischte Pimelinsäureester gebildet.

Bei Basenüberschuß und längerer Reaktionszeit kann allerdings unter Entfernung des leichtersiedenden Alkohols der symmetrische Diester des höheren Alkohols entstehen.

Pro Äquivalent 2-Oxocyclohexancarbonsäureester bzw. dessen Salz kommen 1 - 20 Äquivalente Alkohol zum Einsatz.
Als Base wird vorzugsweise das feste Alkalialkoholat des einzusetzenden Alkohols oder seine alkoholische Lösung in einer Menge von 1 - 3 Äquivalenten pro Äquivalent 2-Oxocyclohexancarbonsäure verwendet.

Die Reaktionen nach Gleichung I und II können in semi-batch-Fahrweise in einem Reaktionsgefäß nacheinander oder auch im semikontinuierlichen Betrieb ausgeführt werden.

Die Aufarbeitung wird üblicherweise durch Zugabe einer Säure eingeleitet. Die Säure kann eine Mineralsäure, wie z. B. HCl, H₂SO₄, HNO₃ oder H₃PO₄ sein, aber auch eine organische Säure, wie z. B. Ameisensäure, Essigsäure, Propionsäure, Buttersäure oder eine Fettsäure, wie z. B. 2-Ethylhexansäure.

Der protonierte Pimelinsäureester kann sehr leicht durch Destillation aus dem Gemisch nach der sauren Aufarbeitung durch Destillation gewonnen werden.

### Beispiel 1

In einem 1-l-Rührkolben werden bei Raumtemperatur 236 g (2 mol) Diethylcarbonat und 136 g (2 mol) Natriumethylat pulverförmig mit 200 g Toluol vorgelegt und auf 40 °C erwärmt. Innerhalb von 2 h werden 147 g (1,5 mol) Cyclohexanon zudosiert. Danach werden 92 g (2 mol) Ethanol zugegeben und 12 h am Rückfluß gekocht. Anschließend werden 500 ml 10 %ige H₂SO₄ zugeführt, wobei sich zwei Phasen ergeben, die gut voneinander trennbar sind. Die wäßrige Phase wird mit wenig Toluol nachextrahiert. Die organischen Phasen werden vereinigt und destilliert. Als Hauptprodukt werden 282 g Pimelinsäurediethylester erhalten (Kp 178 °C/40 mbar), was einer Ausbeute von 87 % entspricht. Nichtumgesetztes Diethylcarbonat und Toluol können zurückgeführt werden.

### Beispiel 2

Es werden bei Raumtemperatur 180 g (2 mol) Dimethylcarbonat und 108 g (2 mol) Natriummethylat pulverförmig sowie 200 g Toluol vorgelegt. Bei 40 °C werden zu diesem Gemisch innerhalb von 2 h 137 g (1,4 mol) Cyclohexanon zudosiert. Danach werden 96 g (3 mol) Methanol hinzugefügt. In einem Druckgefäß wird dieses Gemisch 1 h bei 110 °C gerührt (bei etwa 3 bar).

Zur Aufarbeitung werden 500 ml 10 %ige H₃PO₄ zugegeben. Nach Phasentrennung und Nachextraktion der wäßrigen Phase mit 100 ml Toluol werden die organischen Phasen vereinigt und destilliert. Als Hauptprodukt werden 224 g Pimelinsäuredimethylester (Kp 134 °C/20 mbar) erhalten. Das entspricht einer Ausbeute von 85 % der Theorie.

### Beispiel 3

In einem Glaskolben mit Rückflußkühler und Rührer werden 236 g (2 mol) Diethylcarbonat, 136 g (2 mol) Natriumethylat pulverförmig und 200 g Anisol vorgelegt. Innerhalb von 3 h werden bei 40 °C 147 g (1,5 mol) Cyclohexanon zudosiert. Im Anschluß an diese Reaktionszeit werden 148 g (2 mol) Butanol-1 hinzugefügt. Das Gemisch wird nun auf 120 °C erwärmt, wobei sich Siederückfluß einstellt. Nach 1 h wird das Gemisch abgekühlt und mit 500 ml 10 %iger H₃PO₄ aufgearbeitet. Die organische Phase wird im Vakuum destilliert. Als Hauptprodukt werden 285 g Pimelinsäurebutylethylester erhalten (Kp 178 °C/25 mbar). Das entspricht einer Ausbeute von 78 % der Theorie.

### Beispiel 4

In einem 2-1-Glaskolben mit Rührer werden 270 g (3 mol) Dimethylcarbonat, 162 g (3 mol) NaOCH₃ pulverförmig und 300 g 4-tert.-Butyltoluolcyclohexanon vorgelegt. Bei 50 °C werden innerhalb von 3 h 231 g (1,5 mol) 4-tert.-Butylcyclohexanon zudosiert. Anschließend wird das Gemisch in einem Druckgefäß mit 300 g Methanol auf 120 °C erwärmt und 1 h gerührt.

Danach werden 500 ml 10 %ige H₂SO₄ zugefügt. Es bilden sich zwei gut trennbare Phasen. Die wäßrige Phase wird mit wenig 4-tert.-Butyltoluol nachextrahiert. Die organischen Phasen werden nun destilliert, wobei im Hauptlauf 244 g 4-tert.-Butylpimelinsäuredimethylester erhalten werden. Die Ausbeute beträgt 86 % der Theorie.

### Beispiel 5

In einem Glaskolben mit Rührer werden 180 g (2 mol) Dimethylcarbonat, 330 g (2 mol) 30 %ige NaOCH₃-Lösung, 200 g Anisol und 180 g Butanol-1 vorgelegt. Das Gemisch wird 3 h am Rückfluß gekocht. Danach wird das überschüssige Methanol abdestilliert. Nachdem sich weitgehend eine Umesterung des Dimethyl- zum Dibutylcarbonat vollzogen hat, werden nun bei 70 °C innerhalb von 2 h 147 g (1,5 mol) Cyclohexanon zudosiert. Danach werden weitere 200 g Butanol-1 hinzugefügt und bei ca. 120 °C am Rückfluß gekocht.

Anschließend wird mit 1 000 ml 10 %iger H₃PO₄ aufgearbeitet. Nach Phasentrennung wird die wäßrige Phase destilliert. Als Hauptprodukt werden 310 g Pimelinsäuredibutylester erhalten, was einer Ausbeute von 76 % der Theorie entspricht.

### Beispiel 6

In einem Reaktionsgefäß mit Rührer werden 348 g (2 mol) Di-n-butylcarbonat und 140 g (2 mol) KOCH₃-Pulver in Gegenwart von 500 ml 4-tert.-Butyltoluol vorgelegt.
Bei 40 °C werden innerhalb von 4 h 154 g (1 mol) 4-tert.-Butylcyclohexanon zudosiert.
Anschließend wird das Gemisch mit 400 g n-Butanol 3 h bei 110 °C gerührt. Danach werden 600 ml 10 %ige HCl zugefügt. Es bilden sich zwei gut trennbare Phase. Die wäßrige Phase wird mehrmals nachextrahiert. Die organischen Phasen werden destilliert, wobei 180 g 4-tert.-Butylpimelinsäuredibutylester erhalten werden, was einer Ausbeute von 74 % der Theorie entspricht.

## Patentansprüche

1. Verfahren zur Herstellung von 0 bis 3 Alkylsubstituenten enthaltenden Pimelinsäureestern aliphatischer oder aromatischer Alkohole,
dadurch gekennzeichnet,
daß in an sich bekannter Weise ein gegebenenfalls alkylsubstituiertes Cyclohexanon zu dem entsprechenden 2-Oxocyclohexancarbonsäureester carboxyliert wird und gleichzeitig oder anschließend in Gegenwart einer starken Base das entsprechende Salz gebildet wird, welches mit einem Alkohol bei Temperaturen von 50 - 250 °C unter Ringöffnung umgesetzt wird.

2. Verfahren nach Anspruch 1,
dadurch gekennzeichnet,
daß aliphatische, verzweigte oder unverzweigte Alkohole mit 1 bis 10 C-Atomen, vorzugsweise 1 bis 3 C-Atomen, oder Alkohole von Araliphaten mit 7 bis 12 C-Atomen eingesetzt werden.

3. Verfahren nach den Ansprüchen 1 und 2,
dadurch gekennzeichnet,
daß pro Äquivalent 2-Oxocyclohexancarbonsäureester bzw. dessen Salze, 1 bis 20 Äquivalente Alkohol eingesetzt werden.

4. Verfahren nach den Ansprüchen 1 bis 3,
dadurch gekennzeichnet,
daß bei der Umsetzung als Base ein Alkalialkoholat, vorzugsweise das des eingesetzten Alkohols, in einer Menge von 1 bis 3 Äquivalenten pro Äquivalent 2-Oxocyclohexancarbonsäureester verwendet wird.

5. Verfahren nach den Ansprüchen 1 bis 4,
dadurch gekennzeichnet,
daß die Alkylsubstituenten des Pimelinsäureesters C₁-C₆-KW-Reste sind.

6. Verfahren nach den Ansprüchen 1 bis 5,
dadurch gekennzeichnet,
daß die Reaktion oberhalb des Siedepunktes der entsprechenden Alkohole auch unter Druck ausgeführt wird.

7. Verfahren nach den Ansprüchen 1 bis 6,
dadurch gekennzeichnet,
daß die Base vorzugsweise fest oder in Form einer alkoholischen Alkalialkoholatlösung unter den Reaktionsbedingungen gemäß der Erfindung portionsweise oder kontinuierlich zugefügt wird.

8. Verfahren zur Herstellung von Pimelinsäureestern aliphatischer oder araliphatischer Alkohole nach Anspruch 1,
dadurch gekennzeichnet,
daß in einer ersten Reaktionsstufe Cyclohexanon mit Dialkylcarbonat mit einer starken Base, vorzugsweise mit Alkalialkoholat, zu dem Salz des 2-Oxocyclohexancarbonsäureesters umgesetzt und vor der weiteren Behandlung nicht isoliert wird.

9. Verfahren nach den Ansprüchen 1 bis 8,
dadurch gekennzeichnet,
daß die Reaktion auch in Gegenwart eines Lösungsmittels, vorzugsweise aromatischen, aliphatischen oder alicyclischen Kohlenwasserstoffen, aliphatischen oder aromatisch substituierten Ethern, Fetttalkoholen und cyclischen Kohlensäureestern durchgeführt wird.

## Revendications

1. Procédé de fabrication d'esters d'acide pimélique contenant de 0 à 3 substituants alkyle, avec des alcools aliphatiques ou aromatiques,
caractérisé en ce qu'
on carboxyle d'une manière connue en soi une cyclohexanone éventuellement substituée par un alkyle en l'ester correspondant de l'acide 2-oxocyclohexanecarboxylique et en même temps ou ensuite on forme en présence d'une base forte le sel correspondant qui est mis en réaction avec ouverture du cycle avec un alcool à des températures allant de 50 à 250°C.

2. Procédé selon la revendication 1,
caractérisé en ce qu'
on met en oeuvre des alcools aliphatiques, ramifiés ou non ramifiés ayant de 1 à 10 atomes de carbone de préférence de 1 à 3 atomes de carbone, ou des alcools d'arylaliphatiques ayant de 7 à 12 atomes de carbone.

3. Procédé selon les revendications 1 et 2,
caractérisé en ce qu'
on met en oeuvre par équivalent d'ester d'acide 2-oxocyclohexane carboxylique ou de leurs sels de 1 à 20 équivalents d'alcool.

4. Procédé selon les revendication 1 à 3,
caractérisé en ce qu'
on utilise lors de la réaction, comme base, un alcoolate de métal alcalin, de préférence celui de l'alcool mis en oeuvre, en une quantité allant de 1 à 3 équivalents par équivalent d'ester d'acide 2-oxocyclohexane carboxylique.

5. Procédé selon les revendications 1 à 4,
caractérisé en ce que
les substituants alkyle de l'ester d'acide pimélique sont des radicaux d'hydrocarbure en C₁-C₆.

6. Procédé selon les revendications 1 à 5,
caractérisé en ce qu'
on effectue la réaction au dessus du point d'ébullition des alcools correspondants également sous pression.

7. Procédé selon les revendication 1 à 6,
caractérisé en ce qu'
on ajoute la base de préférence solide ou sous forme d'une solution alcoolique d'alcoolate de métal alcalin dans les conditions de réaction conformément à l'invention, par portions ou d'une manière continue.

8. Procédé de production d'esters d'acide pimélique avec des alcool aliphatiques ou arylaliphatique selon la revendication 1,
caractérisé en ce que
dans une première étape de réaction, on fait réagir la cyclohexanone avec un carbonate de dialkyle en présence d'une base forte, de préférence avec un alcoolate de métal alcalin, en sel d'ester d'acide 2-oxocyclohexane carboxylique et, avant le traitement ultérieur il n'est pas isolé.

9. Procédé selon les revendications 1 à 8,
caractérisé en ce qu'
on effectue la réaction également en présence d'un solvant, de préférence des hydrocarbures aromatiques, aliphatiques, ou alicycliques, des éthers aliphatiques ou substitués par un aromatique, des alcools gras et des esters d'acide carbonique cycliques.

## Claims

1. A process for the preparation of esters of pimelic acid, containing from 0 to 3 alkyl substituents, with aliphatic or aromatic alcohols, characterized in that, in a manner known per se, a cyclohexanone which is substituted, if desired, by alkyl is carboxylated to give the corresponding 2-oxocyclohexanecarboxylic ester and at the same time or subsequently the corresponding salt is formed in the presence of a strong base, the salt being subjected to ring-opening reaction with an alcohol at temperatures of 50 - 250°C.

2. A process according to claim 1, characterized in that aliphatic, branched or unbranched alcohols having 1 to 10 carbon atoms, preferably 1 to 3 carbon atoms, or araliphatic alcohols having 7 to 12 carbon atoms are employed.

3. A process according to either of claims 1 and 2, characterized in that from 1 to 20 equivalents of alcohol are employed per equivalent or 2-oxocyclohexanecarboxylic ester or salts thereof.

4. A process according to any of claims 1 to 3, characterized in that, in the reaction, the base used is an alkali metal alcoholate, preferably that of the alcohol employed, in a quantity of 1 to 3 equivalents per equivalent of 2-oxocyclohexanecarboxylic ester.

5. A process according to any of claims 1 to 4, characterized in that the alkyl substituents of the pimelic ester are C₁-C₆ hydrocarbon radicals.

6. A process according to any of claims 1 to 5, characterized in that the reaction is carried out above the boiling point of the corresponding alcohols, under pressure if desired.

7. A process according to any of claims 1 to 6, characterized in that the base is added, in portions or continuously, preferably in solid form or in the form of an alcoholic solution of alkali metal alcoholate under the reaction conditions according to the invention.

8. A process for the preparation of pimelic esters of aliphatic or araliphatic alcohols according to claim 1, characterized in that, in a first reaction step, cyclohexanone is reacted with dialkyl carbonate using a strong base, preferably using alkali metal alcoholate, to give the salt of the 2-oxocyclohexanecarboxylic ester, which salt is not isolated before further treatment.

9. A process according to any of claims 1 to 8, characterized in that the reaction is carried out also in the presence of a solvent, preferably aromatic, aliphatic or alicyclic hydrocarbons, aliphatic or aromatically substituted ethers, fatty alcohols and cyclic carbonates.
